# EUROPEAN PATENT APPLICATION

(11) **EP 4 338 698 A1**
(43) Date of publication of application: **20.03.2024**
(21) Application number: 22315210.9
(22) Date of filing: 13.09.2022
(51) Int. Cl.: A61B 90/00, A61B 34/00

(54) **MEDICAL IMAGE PROCESSING APPARATUS FOR GENERATING A DYNAMIC IMAGE OF A PATIENT**

(71) Applicant: Caranx Medical SAS, 75013 Paris (FR)
(72) Inventor: BERTHET-RAYNE, Pierre, 06800 Cagnes-sur-Mer (FR); BECHAR, Ikhlef, 06410 Biot (FR); MIRA, Anna, 06000 Nice (FR); DAMERJIAN PIETERS, Vera, 06140 Vence (FR); PRZYBYLSKI, Flavie, 06800 Cagnes-sur-Mer (FR); MIRCEA, Moscu, 06100 Nice France (FR)
(74) Representative: Hepp Wenger Ryffel AG

(57) **Abstract**

An apparatus (10) for generating a dynamic image of a patient (70) comprising a medical imaging device (20) for generating first dynamic image information of at least a target area of a patient; and a processing device (40) configured to generate augmented imaging information (401) by combining the first image information with second information from a source (30) different from the medical imaging device.

## Description

The present invention relates to an apparatus and a method of. use for generating a dynamic image of a patient, or a portion of a patient.

Image-assisted medical procedures have become standard practice for most surgical interventions, helping surgeons to perform safer and less invasive procedures. The real-time visualization of the surgical field provides surgeons with a better control of the procedure and reduces the risk of errors or accidental injury to the patient.

Ultrasound imaging is widely used in the medical field, notably for instrument guidance, and uses soundwaves to produce a real-time 2D image of a portion of a patient's anatomy on a screen in the surgical theatre.

In recent years, augmented reality imaging has largely progressed to allow the use of such technologies in the medical field (van de Giessen et al. 2009 ; Farshad-Amacker et al. 2020) for generating and displaying a three dimensional view of the patient's anatomy.

Currently, ultrasound imaging is used in augmented reality to produce real-time images of a patient. However, ultrasound imaging is still very challenging for an operator due to the type of images produced as well as the difficulty of navigating an instrument whilst looking at a screen and thus requires a steep learning curve to master. The quality of ultrasound images is also easily affected by motion of, or within, the patient or motion of the ultrasound device. This can easily result in inaccurate images and difficulties during a procedure.

It is an object of the present invention to overcome the drawbacks of the prior art and in particular at least one of the above-mentioned disadvantages of the prior art. In particular, the apparatus and method according to the present invention shall promote fast and reliable imaging an improved surgical guiding allowing improved patient safety during procedures. The invention can be particularly used for a partially or fully automated TAVI (transcatheter aortic valve implantation) procedure.

These and other objects are solved with an apparatus and a method according to the independent claims.

In a first aspect, the invention provides an apparatus adapted to generate a dynamic image of a patient, optionally a dynamic image of a portion of a patient. The apparatus may comprise a non-ionizing imaging device. The imaging device may be configured to generate first dynamic image information of a patient, optionally at least a target area of a patient. The apparatus may further have a processing device configured to generate augmented imaging information by combining the first image information with second information from a source different from the imaging device. The second information may be associated with an instrument used during a procedure.

Additionally or alternatively, the second information may be associated with the patient's anatomy.

The imaging device may be configured to capture real-time images and information of a patient, optionally of a target area of a patient.

Real-time imaging refers to live images that may be taken by the Apparatus and/or by a skilled practitioner.

The apparatus may allow for ease of use by practitioners with real-time feedback of in-vivo conditions.

The non-ionising imaging device may be any type of imaging device that does not emit ionising radiations. For example, the imaging device may be any one or more of: an ultrasound unit; and magnetic resonance imaging unit (MRI); an optical imaging unit; a medical grade camera.

The processing device may be connected to one or more image information sources for receiving image information from the one or more image information sources. A first image source may be for example the medical imaging device. A second image information source may be a source different from the first image information source. The second information source may provide information relative to an instrument used during the procedure. Additionally or optionally, the second information source may provide information relative to the patient's anatomy.

The processing device may extract information from one or more information sources to generate augmented image information. The processing device may extract the information from one or more information source by use of one or more algorithms.

Providing the apparatus with a non-ionising imaging unit may avoid the risks of exposing the patient or the practitioner to ionising radiation. Exposure to ionising radiation, especially over prolonged periods of time, is known to have adverse effects on the human body such as damaging DNA molecules and thus, inducing cancers such as Leukaemia.

Generating a dynamic image from combined image information issued from different sources may allow for improved imaging view of the surgical field, for example by improving quality and reducing the chances of noise in the medical imaging. This in turn may allow for better patient outcomes. It is e.g. possible to combine information from two or more image modalities. For example add low resolution details based on a CT scan can be added to a to high resolution US image (the inverse is also viable ) or 3D anatomical details from a CT image can be added to a 2D ultrasound image.

In some embodiments the apparatus may be configured for dynamic image-assisted medical instrument guidance within a patient.

The medical imaging device may use non-magnetic-field based imaging.

The non-magnetic-field based imaging device may be any imaging devices that does not emit a magnetic field such as ultrasound devices, optical imaging devices, thermal imaging devices, or medical grade cameras.

Optionally the medical imaging device may be adaptable for autonomous operation; semi-autonomous operation; operation by a trained practitioner.

In some embodiments the medical imaging device may be an ultrasound device.

The ultra-sound device may be configured to produce multi-dimensional sonograms of the patient or a portion of the patient in real-time. For example, the ultrasound device may produce live two-dimensional (2D) images; three-dimensional (3D) images; four-dimensional (i.e. time dependent 3D images) images; High-definition (HD) images. Optionally, the ultrasound device may be configured to produces a plurality of sonograms in different dimensions at a same time.

As ultrasound devices are relatively compact the use of an ultra-sound device may avoid the use of overly encumbering imaging devices and may thus be easier for a practitioner to use. Additionally, using an ultra-sound device may reduce the exposure of the practitioner and patient to potentially harmful radiation.

In some embodiments the medical imaging device may comprise a computing unit adapted to reconstruct a three-dimensional image.

Optionally, the computing unit may be adapted to reconstruct one or more multi-dimensional images. For example, the reconstructed image may be a 4D or HD image.

The reconstruction of a three-dimensional, or multi-dimensional, image of a patient or of a portion of a patient may allow for better visualisation of the intracorporeal disposition of a patient. This in turn may allow for improved navigation of an instrument within a patient as well as reducing the risk of errors due to lack of visibility or poor image quality.

In some embodiments the apparatus may further comprise a display device. The display device may be configured to generate a visual display of the augmented imaging information.

The display device may be configured to display a reconstructed multi-dimensional image, for example a three-dimensional image reconstructed by the computing unit.

The dynamic image may be a muti-dimensional image of the patient or of a portion of the patient. The multi-dimensional image may be a reconstructed image formed from a combination of multiple images issued from a plurality of sources.

The resolution of the reconstructed image may be set and/or changed during the procedure. The resolution may be changed in an autonomous and/or automatic manner. Additionally or alternatively the resolution may be changed manually by a skilled practitioner.

A change in resolution may be for example, passing from a view of the full anatomy of a patient to a zoomed-in view of a portion of a patient such as a target zone and/or structure.

The display device may be freely moveable over the working space. Optionally the display device may be connected to a mount which is fixed with respect to the working space, e.g. connected by means of a robotic arm.

Optionally the display device may be selectively moved or positioned autonomously; semi-autonomously; manually.

The working space refers to an area adjacent to the patient, or a portion of the patient, in which a component of the apparatus is operated. For example, the working space may be the surgical field and/or the operating table.

The display may be at least partly connected to and/or aligned with the medical imaging unit.

Providing the apparatus with a moveable display device allows for augmented imaging of target zones of a patient. A moveable display device may further allow the display device to follow the movements of the medical imaging device.

The display device may optionally be at least one of the following: a three-dimensional display device; a holographic display device; an image projector or a head-worn display device. Additionally or alternatively, the display device may be a high-definition and/or multi-dimensional display device.

In some embodiments the display device may comprise a projector. The projector may be configured to project an image into a physical working space. Optionally, the image may be projected into a physical working space in which the patient is located.

The image may be projected in a manner so that it is at least partly adjacent to a patient or a target zone of the patient.

Additionally or alternatively, the projected image may be projected in a manner so as to be at least partly superposed with the patient or a target zone of the patient. The alignment between the patient/workspace and projections can be achieved by using e.g. fiducials or computer-vision-aided landmark detection.

Further additionally or alternatively, the projected image may be at least partly adjacent and/or aligned with the medical imaging device. For example, the projected image may be at least partly adjacent to the beam of the ultra-sound device, if provided.

The physical working space may be any space where the apparatus or an element of the apparatus may come, at least partly, into contact with the patient's body.

The physical working space may optionally be any space adjacent to a patient where the medical imaging unit is operated to generate first dynamic image information.

The projector may allow for a see-through-like viewing of the patient and may further allow a better visualisation of an instrument within a patient. This in turn may facilitate the procedure and reduce the risk of errors.

In some embodiments the processing device may be configured to generate information relating to a patient or relating to one or more portions of a patient.

The generated information may pertain to anatomical features of a patient and/or the localisation of said features.

Additionally or alternatively, the generated information may pertain to instruments within a patient and/or the localisation of said instruments within a patient.

The processing device may be configured to generate information relating to one or more of:
- a centreline within a body lumen;
- one or more walls of a body lumen;
- a junction in a body lumen;
- obstructions in a body lumen;
- calcification within a body lumen;
- a shape of a body lumen;
- contact force and/or pressure against a body lumen;
- contact force and/or pressure against an instrument;
- deformation of the body in contact with an instrument;
- a position of an instrument within a body lumen;
- a native or artificial valve within a body lumen;
- navigation of an instrument within a body lumen;
- introduction of an instrument into a body lumen;
- incision by an instrument;
- an offset between an instrument and a centreline of a body lumen.

In some embodiments the processing device may be configured to generate information relating to one or more of:
- one or more walls of a vascular lumen;
- a junction in a vascular lumen;
- a shape of a vascular lumen;
- one or more singular zones of a vascular lumen, e.g. extremities of a vascular lumen;
- one or more target areas for a medical instrument, e.g. a needle, on a vascular wall and/or lumen.

The information generated by the processing device may be generated based on second information from a source different from the imaging device, e.g. pre-procedural imaging; patient anatomy; information associated with an instrument.

Additionally or alternatively, the information may represent any one, or a selection of: the size; the angulation; the volume of an anatomical feature.

Additionally the information may represent any one, or a selection of: blood flow; blood pressure; cardiac rate; cardiac cycle of the patient.

The generated information may be presented in in any way suitable. For example, the blood pressure may be presented in form of a numerical value.

The processing device may for example generate information concerning the structure and/or delimitations of an anatomical feature. The processing device may further generate a visual display of said structure and/or delimitations in an augmented manner such that the structure and/or delimitations are clearly distinguishable from the surrounding environment.

Generating information relative to any one of the above-listed features may help provide navigational information to assist the practitioner during the procedure. It may also facilitate an at least partly autonomous procedure and/or the suggestion of an instrument pathway.

The processing device may further be configured to generate information using pre-computed images of the patient. Optionally, the generated information may be projected onto the visual display and/or into the physical working space.

The generated information based on pre-computed images may be information relative to the anatomical structure of a patient, e.g. shape metrics of the vascular lumen.

For example, the generated information may be the length and or the diameter of the vascular lumen.

The pre-computed images may be generated based on any previously acquired biological imaging of the patient. For example, the images may come from pre-acquired medical imaging such as, but not restricted to, computed tomography scans (CT scan); magnetic resonance imaging scans (MRI scans); sonograms; X-ray images; positron emission tomography scans (PET scans); Gamma scans.

Using pre-computed images may help improve the quality of the visual display and may allow for improved localization of anatomical features.

The pre-computed images can be projected into the working space and combined with a second source of information by registration, e.g by rigid registration, free-form registration based on landmarks, point cloud or image features according to the information source.

In some embodiments, the processing device may be configured to generate information relating to a contact force and/or pressure against a body lumen. The strain and/or stress on anatomical structures of the patient such as tissue / vessels can be estimated by the means of elastography and biomechanical models of living tissue.

Additionally or alternatively, the processing device may be configured to generate information relating a contact force and/or pressure against an instrument.

The processing device may be adapted to generate information when an instrument comes into contact with a patient or a target zone of a patient.

The processing device may be configured to receive a signal from the instrument. Additionally, the processing device may be adapted to derive information from the received signal.

The instrument may comprise one or more sensors for generating an input signal. For example, the sensors may be pressure sensors for determining the pressure or contact force against a body lumen. For further example the sensors may be position sensors for determining the position of the instrument with regards to the patient or with regards to a target zone of the patient.

The instrument may allow the partitioner to have a real-time reading of the patient's health statistics or statistics concerning the environment in which the instrument is positioned. This in turn may allow for improved puncture of a target zone.

In some embodiments the processing device may be configured to generate warning information about one or more hazard conditions along a lumen route.

The processing device may be configured to generate a warning information based on a hazardous condition.

Hazard conditions may be anything susceptible of constituting an obstacle and/or a feature that may impede navigation along the lumen route.

A hazard condition may further be the incorrect and/or potentially harmful positioning of an instrument within a patient.

In other words, hazardous conditions may be considered as something that may hinder the progression of an instrument within a patient lumen and/or that may induce injury to the patient.
For example, calcifications along a vascular lumen may be considered as a hazard as they may obstruct the passage of an instrument.

Information concerning a hazard condition may be generated based on the second information. Optionally information concerning a hazard condition may be generated based on the first image information.

For example, calcifications of the vascular system may be considered as hazardous. If calcifications are detected based on the second information, e.g. pre-computed images, the apparatus may generate a warning information.

Additionally or alternatively, the processing device may generate warning information based on the information derived from the instrument sensors. For example, the processing device may be adapted to generate information concerning the incorrect angulation of an instrument.

The warning information may be presented in the form of a sensory indication. The sensory indication may be one or more of visual; auditory; haptic.

The warning information may help reduce the risk of problems arising during a procedure as they alert the practitioner to Hazard.

In some embodiments the processing device may be configured to generate navigation guidance with respect to a lumen route to a target location.

The processing device may be adapted to use algorithms and/or artificial intelligence and/or machine learning techniques to generate navigation guidance. This can be achieved by using pre-operative data, such as available CT scans.

The generated navigation guidance may be relative to the position of an instrument used during a procedure. For example, the navigation guidance may propose an improved position and/or orientation and/or shape of the instrument with regards to the vessel walls and/or with regards to a hazardous condition.

Additionally or alternatively, the navigation guidance may, amongst others, provide a suggested navigation pathway for the instrument. The pathway may change dynamically as the instrument advances to account for patient motion or deformation induced by interaction between the instrument and the body. The suggested pathway may run at least a part of the way from an incision point to a target point.

The dynamic navigation paths may be computed using free-form deformation of solid geometric models. The local deformation field may be estimated by using free-form registration of two sources of information based on local anatomical features.

The incision point may be a suggested and/or pre-decided incision point. The incision point may be a point on the patient determined to be optimal for instrument incision for improved procedural success. The incision point may be generated by the apparatus based on the first imaging information source and/or the second information source. In particular, it may be dynamically updated/adjusted with a higher precision with the second information source.

The target point may be the zone of the patient that is targeted during the procedure. For example, the target point may be a cardiac valve if the apparatus is used in a transcatheter aortic valve implantation (TAVI) procedure.

The navigation guidance may facilitate an at least partly autonomous procedure. It may also assist the practitioner during the procedure.

Additionally or alternatively, the navigation guidance may be presented in the form of a sensory indication. The sensory indication may be visual and/or auditory and/or haptic.

In some embodiments the medical imaging device may be an extracorporeal device.

The medical imaging device may be adapted to move over the working space.

The medical imaging device may be configured to move autonomously over the working space or may be at least partly manipulated by a trained practitioner.

Optionally, the medical imaging device may be fixedly or removably connected to at least a mount fixedly arranged within the part of the working space.

In some embodiments the medical imaging device is at least one selected from: an intracorporeal device; an endoluminal device; an endovascular device.

Additionally or alternatively, the medical imaging device may be an intraluminal and/or intravascular device.

The medical imaging device may be configured to be positioned and/or move within a patient body lumen for intracorporeal imaging. The medical imaging device may be, for example, an intravascular ultra-sound device.

The intracorporeal device may allow the acquisition of intracorporeal information, such as the structure of anatomical features. For example, the use of an intravascular imaging device may allow the generation of information regarding the shape and size of vascular structures such as the aorta and the aortic annulus.

This feature may help provide the practitioner with useful information concerning the intracorporeal structures of a patient. This may be particularly useful as the body configuration may change during the procedure. Therefore it can be used to adjust the pre-operative configuration. This may improve the navigation of instruments within the patient as instruments will be able to be chosen in according to the specificities of the intracorporeal structures.

Additionally having an apparatus combinable with extracorporeal medical imaging devices and intracorporeal medical imaging devices may allow the apparatus to be adapted to different procedures. Being more versatile, the device may reduce the need for additional apparatus in the operating theatre and thus may reduce the cost of medical equipment necessary for a procedure.

In some embodiments the second information may comprise pre-procedural information obtained prior to a medical procedure.

The pre-procedural information may be any information related to the patient and stored within the apparatus prior to procedure.

The second information may be generated from medical examination and/or imaging of the patient. For example, the information may be relative to anatomical features or vital signs acquired prior to the procedure.

The second information may comprise at least one selected from: x-ray image information; fluoroscopic image information; CT-scan image information; MRI image information; information from an instrument force sensor; information from an instrument pressure sensor; information from a magnetic resonance imaging device.

Using pre-procedural information may allow for improved over-all information presentation during a procedure and may help improve the quality of the displayed visual for guiding a practitioner.

In some embodiments the processing device may comprise an image processing engine. The image processing engine may serve for correlating relative positioning between the first image information and the second information, to effect position registration of the first and second information.

Additionally, the image processing engine may serve for estimating a deformation or/and displacements between the first image information and the second image information.

The processing device may use the correlated first image information and second information to generate a localisation map permitting the practitioner and/or the apparatus to know imaging device and/or instrument position at any given time.

Correlating first image information and second information may facilitate navigation for a practitioner or for the apparatus.

Additionally, the localisation map may allow for device and/or instrument localisation at any moment during the procedure thus facilitating navigation.

Additionally, the apparatus may further comprise at least one instrument insertable at least partly into the body. The insertable instrument may be distinct from the medical imaging device.

For example, the instrument may be any suitable medical instrument. The instrument may be for example one or more of: a needle; an endovascular device; a catheter; a guidewire; an implant.

In some embodiments the apparatus may be adapted to predict the path of an instrument and/or display the predicted target and/or trajectory of the instrument.

In some embodiments the apparatus may further comprise a robotic apparatus for manipulating the instrument.

Additionally or alternatively, the robotic apparatus may manipulate the medical imaging device.

Providing the apparatus with an instrument and/or robotic apparatus may facilitate an at least partly autonomous operation of the apparatus.

In a second aspect the invention provides a method of planning at least a portion of a medical procedure to be at least partly carried out by robot.

Additionally or alternatively, the invention provides a method of planning at least a portion of a medical procedure to be at least partly carried out by a remotely-operated apparatus.

The planning method may comprise a step of operating a medical imaging device comprising a non-ionizing imaging transducer to generate first dynamic image information of a target area of a patient.

The method may further comprise a step of electronically processing the first image information to generate augmented imaging information by combining the first image information with second information from a source different from the medical imaging device, the second information associated with at least one selected from: an instrument to be used during the procedure and/or the patient's anatomy. Preferably, the method is carried out by an apparatus as described hereinabove.

### Brief Description of the Drawings

The invention will be better understood with reference to the following description of preferred embodiments and the accompanying drawings, in which:
- Fig. 1: is a schematic view of an embodiment of an apparatus for generating a dynamic image of a patient.
- Fig. 2: is a schematic view of an second embodiment of an apparatus for generating a dynamic image of a patient.
- Fig. 3: is a schematic view of an third embodiment of an apparatus for generating a dynamic image of a patient.
- Fig. 4: is a schematic view of an fourth embodiment of an apparatus for generating a dynamic image of a patient.
- Fig. 5: is a schematic view of an fifth embodiment of an apparatus for generating a dynamic image of a patient.
- Fig. 6: is a schematic longitudinal cross-section view of a medical imaging component and display component of an apparatus for generating a dynamic image of a patient.
- Fig. 7: is a schematic view of an sixth embodiment of a medical imaging component and display component of an apparatus for generating a dynamic image of a patient.
- Fig. 8: is a schematic view of an embodiment of a generated dynamic image of a patient.
- Fig. 9: is a schematic view of a second embodiment of a generated dynamic image of a patient.
- Fig. 10: is a schematic view of a third embodiment of a generated dynamic image of a patient.
- Fig. 11: is a schematic view of a fourth embodiment of a generated dynamic image of a patient.
- Fig. 12: is a schematic view of a fifth embodiment of a generated dynamic image of a patient.
- Fig. 13: is a schematic view of a sixth embodiment of a generated dynamic image of a patient.
- Fig. 14: is a schematic view of a seventh embodiment of a generated dynamic image of a patient.
- Fig. 15: is a schematic illustration of steps in an example technique for planning a medical procedure.

### Detailed Description of Some Embodiments:

Non-limiting embodiments of the invention are now described by way of example only.

In the illustrations, the same reference numerals are used to denote the same or equivalent features amongst different embodiments and examples. Unless described to the contrary, the description of a feature in one embodiment may also apply to the same or equivalent feature in one embodiment or example. Features may also be interchanged in embodiments as desired.

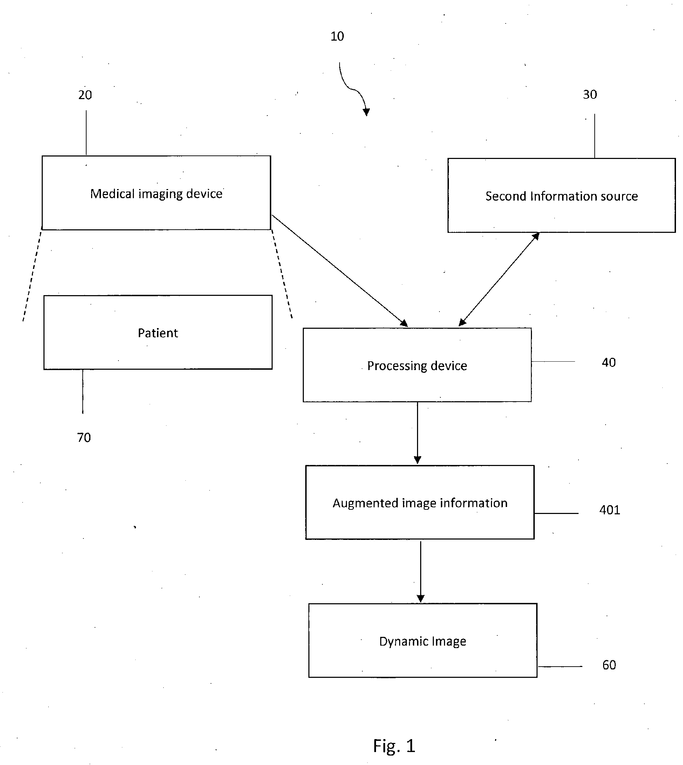

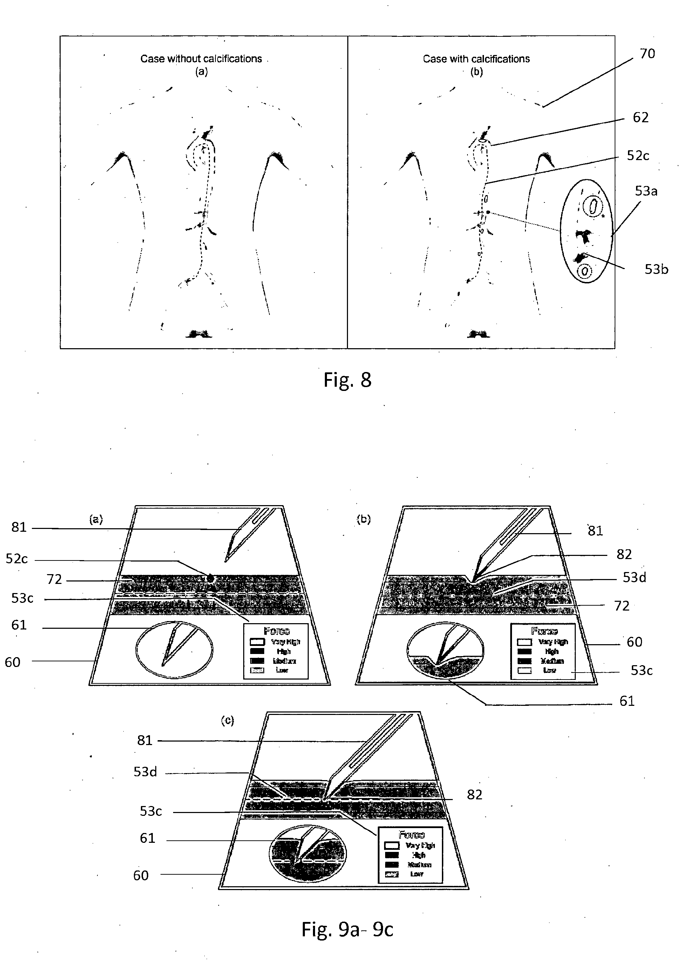

Referring to figure 1, an apparatus 10 configured for generating a dynamic image 60 of a patient 70 is shown. The dynamic image may be of the entirety of the patient 70 or of a portion of the patient 70' (see fig. 9a-c).

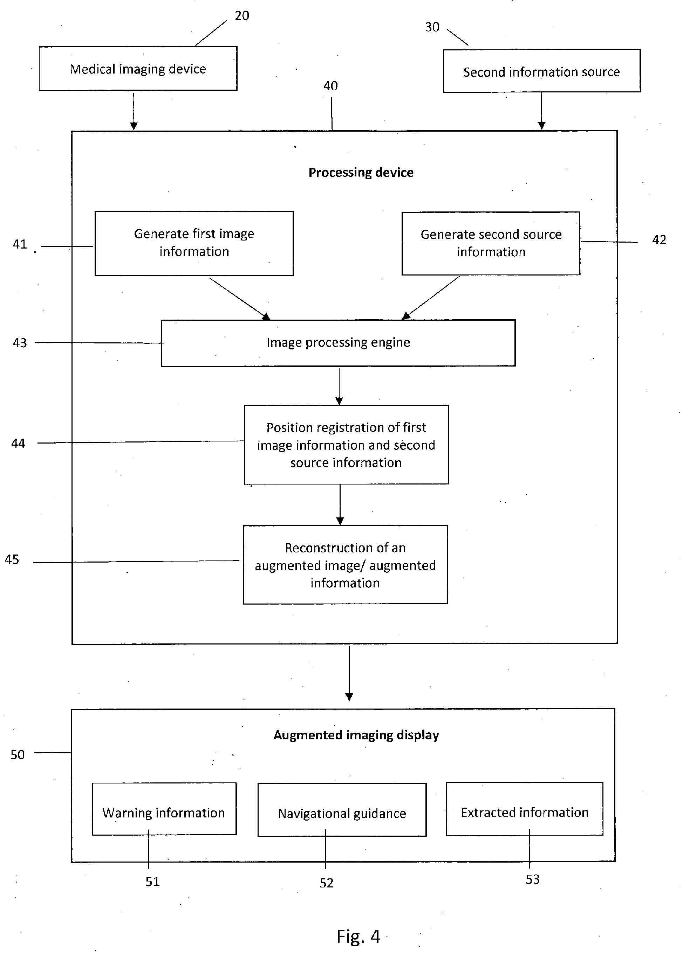

The apparatus comprises a first image information source, for example a medical imaging device 20 (see figure 4) configured to take first images of a patient 70 for generating first image information. The apparatus further comprises a second information source 30. The first and second information source 20,30 are configured to communicate with a processing device 40 of the apparatus.

The processing device 40 is adapted to extract information form the first and second information source 20,30 and combine first and second source information so as to generate augmented imaging information 401.

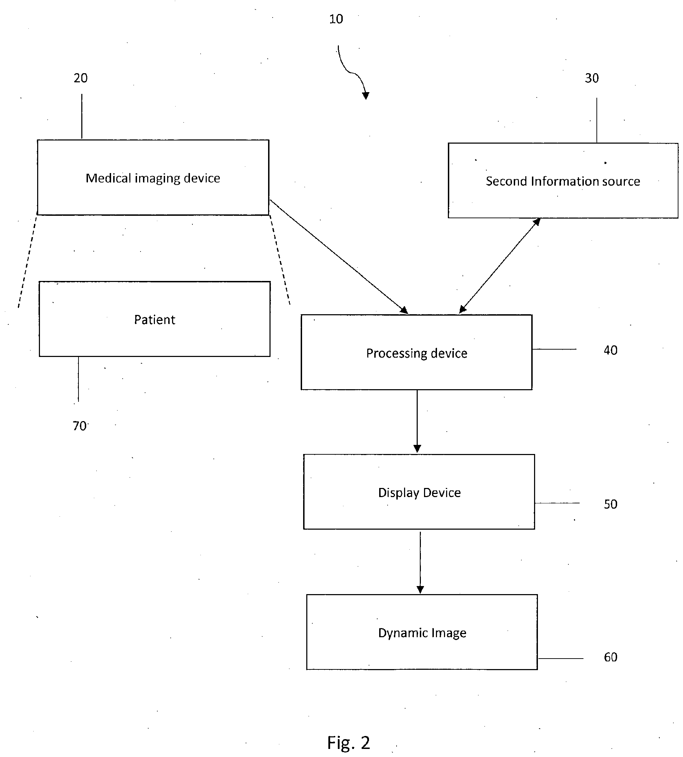

Illustrated in figure 2, the apparatus 10 additionally has a display device 50 for displaying the dynamic image 60 of the augmented imaging information 401.

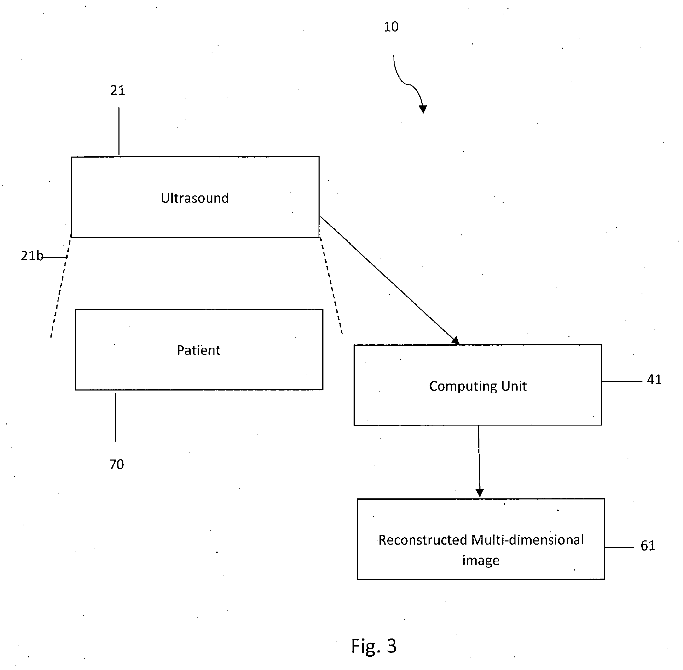

As can be seen in figure 3, in this embodiment the medical imaging device is an ultrasound device 21. For example, the ultrasound device can be an intracorporeal ultrasound device. Optionally the intracorporeal ultrasound device can be mounted on a catheter for incision within the patient. The ultrasound device is connected to the computing unit 41 of the apparatus. The ultrasound device 21 is configured to take dynamic/ live/ real-time images of a patient 70. The beam 21b of the ultrasound device extends over at least a portion of the patient 70 for capturing images of the patient 70.

Some exemplary information that may be extracted from an ultrasound imaging device 21 is aortic root size; number and state (calcified, blocked, functional) of valve leaflets,, aortic annulus size, heart valve size, calcification degree; cardiac cycle phase indicating if the heart valve is opened or closed.

The apparatus has a computing unit 41. The computing unit 41 communicates with the medical imaging device, in this case an ultrasound device 21, and is configured to generate a reconstructed multi-dimensional image 61, for example a three-dimensional image, of the patient 70 or of a portion of the patient.

Referring to figure 4, the processing device 40 communicates with the medical imaging device 20 to generate first image information 41.

The processing unit 40 also communicates with the second information source 30 to generate second source information 42.

The processing device 40 has an image processing engine 43. The image processing engine 43 correlates relative positioning between generated first image information 41 and generated second source information 42 so as to effect position registration 44 of the first and second image information.

The processing device 40 then reconstructs an augmented image with augmented information 45 using the position registration 44 of first and second source information.

The processing device is connected to a display, here an augmented imaging display 50, e.g. a projector; a holographic display device; a three dimensional display device. The augmented imaging 50 display is configured to display the reconstructed augmented image and information 45.

The augmented display displays, at choice, the augmented image with any one or a plurality of: warning information 51; navigational guidance 52 or extracted information 53.

For example, the warning information can be any information pertaining to a hazard condition within the patient. An exemplary hazard condition is calcifications within the vascular system

For example, the navigational guidance can be any information relative to instrument trajectory, for example, projection of a suggested pathway for the instrument. The navigational guidance can be any information relative to a target zone of a patient, e.g. a suggested incision point. The navigational guidance can also be any information relative to instrument positioning, for example, concerning the current localisation of the instrument and/or suggestion of possibly improved position for the instrument.

For example, the extracted information can be any information. relating to a centreline within a body lumen, e.g. the centreline of a vessel may be displayed. The extracted information may relate to one or more walls of a body lumen, e.g. the vessel walls can be displayed in an manner so as to improve the visualisation of the boundaries of the vessel. The extracted information can concern a junction in a body lumen, e.g. bifurcations in the vascular system can be displayed. Information extracted concerning obstructions in a body lumen can be, for example, obstructions reducing blood flow within a vessel. Information concerning calcification within a body lumen may be extracted, e.g. vascular calcification. The extracted information can be relative to a shape of a body lumen, e.g. length; diameter. Extracted information can be relative to contact force and/or pressure against a body lumen, e.g. contact force and/or pressure applied by a medical instrument against a vessel lumen. The extracted information can also be relative to contact force and/or pressure against an instrument, e.g. the contact force and/or pressure applied to an instrument by of a vessel lumen; by blood flow. Information concerning the position of an instrument within a body lumen, e.g. the current location of an instrument, can be extracted. The extracted information may be relative to a native or artificial valve within a body lumen, e.g. number of valve leaflets; state of the valve (open state; closed state). Information concerning the navigation of an instrument within a body lumen can be extracted, e.g. suggested adjustments to the position of the instrument within a vessel.

The extracted information can be information concerning the introduction of an instrument into a body lumen, e.g. a predicted trajectory of the instrument. Information pertaining to the incision by an instrument, e.g. an access point can be extracted. The extracted information can pertain to an offset between an instrument and a centreline of a body lumen, e.g. when the instrument is incorrectly positioned with regards to the centreline of a vessel. Information concerning one or more walls of a vascular lumen, e.g. the aortic walls, can be extracted. Extracted information can pertain to a junction in a vascular lumen, e.g. branches; bifurcations in a vessel can be extracted. The extracted information may relate to the shape of a vascular lumen, e.g. length; diameter; angulation.

This may help facilitate navigation or positioning of the instrument within a body lumen as well as possibly reducing the risk of injury to the body lumen by incorrect positioning of the instrument.

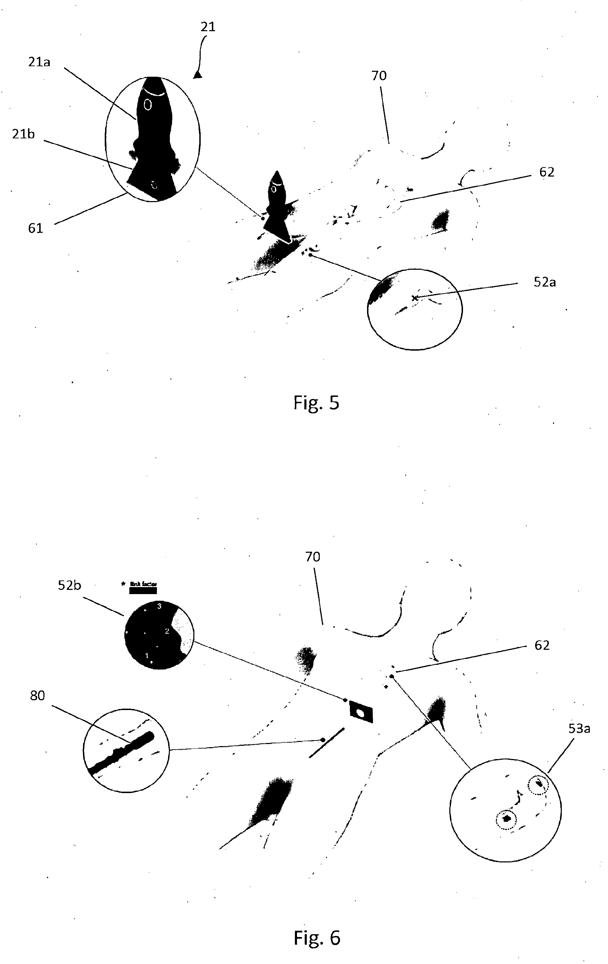

Figure 5 illustrates the use of the medical imaging device 20 of figures 1, 2 and 4. In this example, the medical imaging device 20 is an ultrasound device 21 (see figure 3) having a transducer 21a and a beam 21b positioned over a femoral artery of the patient 70. A first and second augmented image 61, 62 are displayed. The displayed augmented images 61,62 are displayed in the working space, in this case, overlayed on the patient 70 in a see-through-like manner.

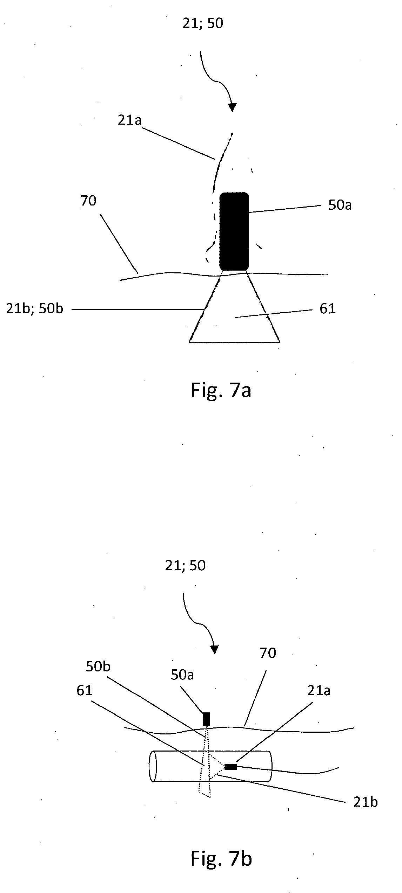

The first augmented image is a localised augmented image 61 of a portion of the patient's 70 vascular system. A display device 50 (see figure 7a) is provided within the transducer 21a of the medical imaging device (see fig. 6) for projecting an augmented image of a portion of the patient 70. The localised augmented image 61 is projected onto the patient 70 in alignment with the beam 21b of the medical imaging device 21.

The second augmented image 61 is an augmented image of part of the patient's 70 vascular system. The augmented image 62 is provided with navigation guidance information pertaining to a suggested incision point 52a for an instrument.

As seen in figure 6, a display of an augmented image 61 of the patient's 70 vascular system structure is projected onto the patient. Calcification 53a can within the vascular system can be seen.

The augmented image displays an augmented three-dimensional representation of an instrument 80 provided by the apparatus. A navigational risk map 52b is further displayed with the augmented image of 61 the patient to facilitate navigation of the instrument 80 within the vascular system of the patient 70. With the elements presented in figures 5 and 6, the apparatus provides a safer and more comprehensive, rapidly adaptable, view of the patient 70. This allows for improved and faster navigation within a patient.

Figures 7a-b illustrate the medical imaging device 20 and display device 50. The medical imaging device 20 is positioned at a target zone of the patient 70 for capturing an image of the target zone. The display device 50 serves to display an augmented image 61 of the target zone.

In figure 7a the medical imaging device 20 is an ultrasound device 21. The display device 50 has a projector 50a housed within the ultrasound transducer 21a. The beam 21b of the ultrasound device 21 and the beam 51b of the display device generated by the projector 50a are superposed with one another.

In figure 7b the display device projector 50a and the ultrasound transducer 21a are separate. The ultrasound device 21 is an intravascular ultrasound device, for example an intravascular ultra-sound device. The display device 50 is an external projector 50a positioned at the target zone of the patient 70.

The ultrasound beam 21b and.the display beam 50b are at least partly perpendicular to one another. The augmented image 61 is displayed at the intersection point of the two beams.

Figure 8 illustrates a displayed augmented image 60 of the patient's 70 vascular structure with a suggested pathway 52c for the instrument during the procedure. The augmented image 62 further displays information concerning the vascular structure. Here calcifications 53a and bifurcations 53b within the vascular system of the patient 70 can be seen. The presence of obstructions/abnormalities for example calcifications, are detected based on pre-computed imaging. The obstructions/abnormalities can also be detected using the medical imaging device, for example the ultrasound device.

The suggested pathway is generated by the apparatus for facilitating the navigation of the instrument during the procedure. To generate a suggested pathway the apparatus considers the hazardous conditions detected within the patient using the first and second information sources as well as considering the extracted structural information of a patient's anatomy, for example the extracted information based on pre-computed imaging and/or information associated with an instrument used during the procedure.

This may allow for less complications during the procedure as well as allowing the choice of adapted instruments in advance of the procedure, thus saving time in the operating theatre.

An apparatus adapted to generate a suggested pathway for the procedure may also allow for an at least partly autonomous procedure.

In figures 9a-c an augmented image 60 of a needle 81 and vessel 72 is displayed. The image 60 is generated by the apparatus. A suggested incision point 52c is also presented along the vessel 72. The image further displays a contact force information 53c between the needle 81 and the vessel wall determined by sensors 82 of the needle 81. The force determined by the sensors 82 is processed by the processing device 40 (see figure 4) to generate the contact force information 53c displayed with the image. The centreline 53d of the vessel 72 is also incorporated into the displayed image.

Additionally, a zoomed-in image 61 of the needle head is displayed.

All these features may help facilitate the navigation of the needle, or more generally an instrument, and may help avoid injury to the patient, for example by helping avoid the needle from completely traversing the vessel.

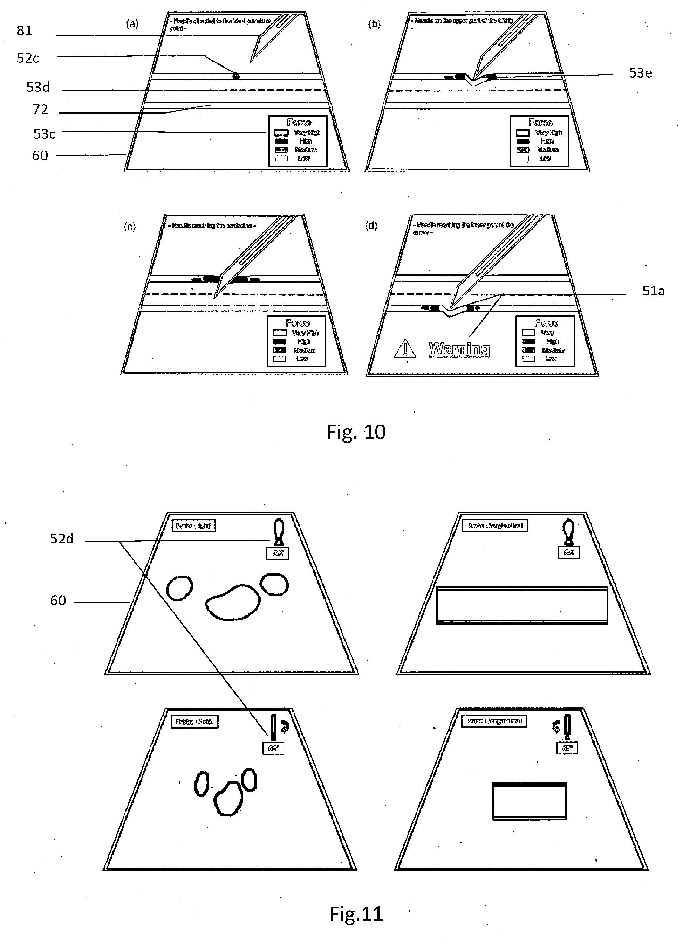

Figures 10 a-d show a displayed augmented image generated by the apparatus 10. The augmented image 60 displays navigational guidance information, such as a suggested incision point 52c for a needle 81 as well as displaying extracted information, for example vessel centreline 53d.

The display further shows generated information relative to the interaction between the needle 81 and the vessel, for example the contact force 53c and deformation of the vessel wall 53e under incisional pressure from the needle.

Additionally, the display of figure 10d showy a warning information 51a displayed to warn of a hazard. In this case the case the hazard is the unintentional traversing of the vessel.

A warning information 51a may also be presented by a visual; auditory and/or sensory alert.

The information may be presented by a change in colour of the displayed structures and/or extracted information of a patient, e.g. a changed colouring of displayed vessel walls.

Additionally or alternatively displayed images of components of the apparatus and/or extracted information relating thereto, may be presented by a change in colouring, e.g. a change in colour of the displayed instrument.

The information may be presented by means of a marking on the displayed augmented image, e.g. to indicate the incision point. The marking may be of any geometrical shape, e.g. a cross, a dot; a triangle; a square.

This feature may allow the apparatus to warn a practitioner in case of an error or a danger thereof. The practitioner may then correct the position and or retract the instrument before unintentionally harming the patient or creating complications to the procedure.

Figures 11 a-d illustrate an augmented image 60 generated by the apparatus, wherein the apparatus has further generated and displayed navigational guidance 52d relative, in this case, to the orientation of the instrument within a vessel. The most optimal probe position is indicated for either an axial or longitudinal view. The displayed indication provides an estimate of the angle in relation to the image content. If the probe is incorrectly positioned, an indication is provided as to how many degrees the user should turn and in which direction. It is also possible to conceive other features such as the placement of the probe on, the vertical axis in order to have a centred image. Furthermore, the force of the probe on the patient can be estimated and indicated..

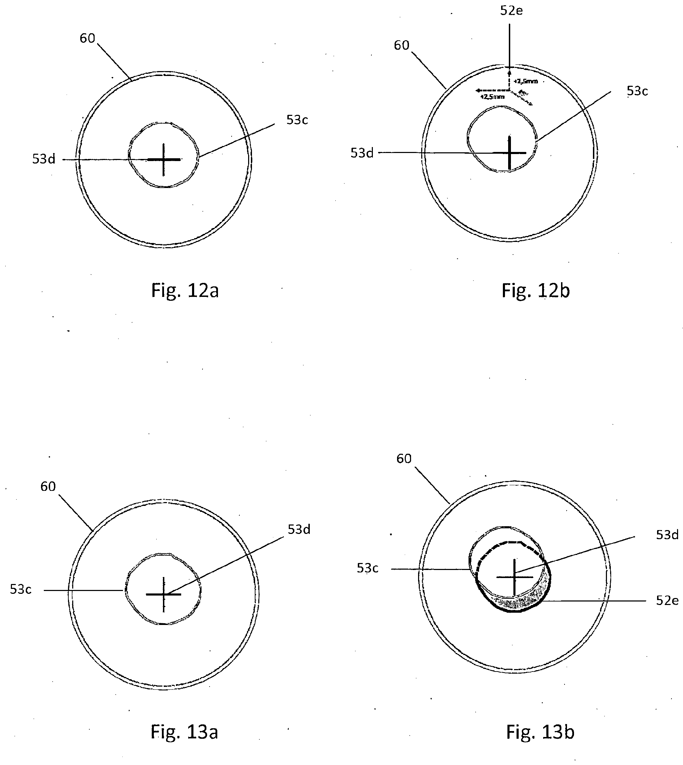

Referring to figures 12a-b an augmented image 60 is shown. The augmented image is provided with structural information 53d and relative instrument positioning as well as navigational guidance information 52e.

In figure 12a the radial position(i.e. with respect to the axis of the instrument 53c) is depicted by a circle relative to the centreline of the structure 53d.

In figure 12b the radial position of the instrument 53c is offcentre with regards to the centreline 53d of the structure. Navigational guidance information 52e is displayed, for example, by directional arrows and distance, to guide the repositioning of the instrument into a preferred positioning.

In figures 13a-b an augmented image 60 is shown. The augmented image 60 is provided with structural information 53d and relative instrument positioning as well as navigational guidance information 52e.

In figure 13a the radial position of the instrument 53c is depicted by a circular ring relative to the centreline of the structure 53d.

Tin figure 13b the radial position of the instrument 53c is offcentre with regards to the centreline 53d of the structure. Navigational guidance information 52e is displayed in this case by a second circle, optionally in a different colour to the first, to guide the repositioning of the instrument into a preferred positioning.

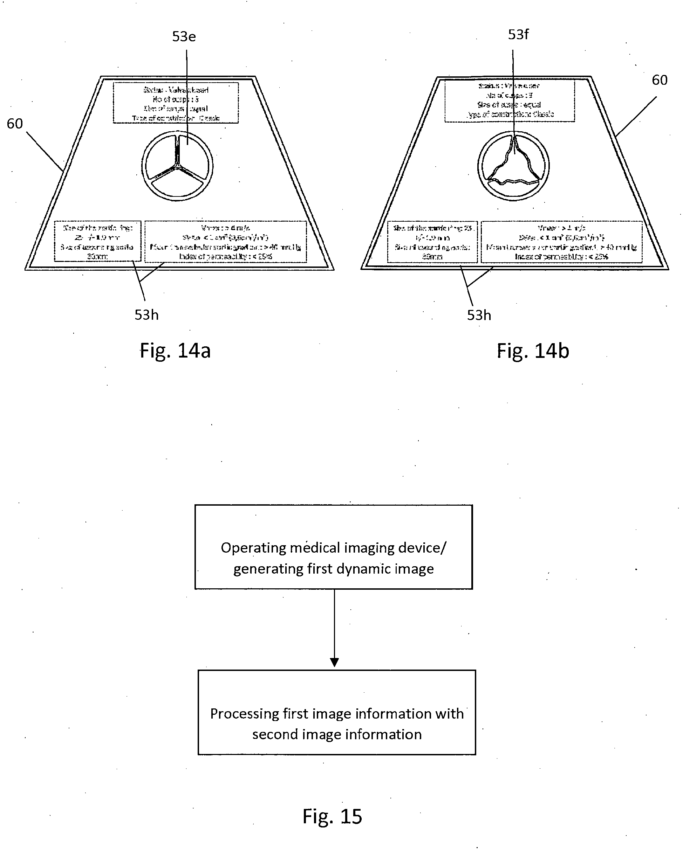

Figures 14a-b illustrate an augmented image 60 of a patient's valve and displaying augmented information 53e; 53f; 53h, relative to the state of the patient's aortic valve.

In figure 14a the augmented image shows the valve in a closed state 53e. Further displayed is structural information and patient health statistics, for example aortic arch measurement; ventricular ejection fraction; maximum transvalvular aortic velocity and others determined by means information extracted from pre-computed imaging and/or a medical imaging device.

Figure 14b shows the patient's valve in an open state 53f.

Being able to see the state of the valve may help with the positioning of ventricular implants. This may avoid trying to cross the valve at the wrong time thus leading to less complications during such procedures.

Figure 15 shows the steps of a method for planning a medical procedure carried out by the apparatus 10.

The first step of such a method consists in operating a medical imaging device to generate first image information of a target area of a patient.

The first image information is then electronically processed and combined with second source information to generate augmented imaging information.

## Claims

1. Medical image processing apparatus (10) for generating a dynamic image of a portion of a patient (70), comprising:
a medical imaging device (20) comprising a non-ionizing imaging unit, for generating first dynamic image information of at least a target area of a patient (70); and
a processing device (40) configured to generate augmented imaging information (401) by combining the first image information with second information from a source (30) different from the medical imaging device, the second information associated with at least one selected from: an instrument (80) used during a procedure and the patient's anatomy.

2. Apparatus according to claim 1, wherein the medical imaging device (20) uses non-magnetic-field based imaging.

3. Apparatus according to claim 1 or 2, wherein the medical imaging device is an ultra-sound device (21).

4. Apparatus according to claim 1, 2 or 3, wherein the medical imaging device (20) comprises a computing unit (41) adapted to reconstruct a three-dimensional image (61).

5. Apparatus according to any preceding claim, further comprising a display device (50) configured to generate a visual display (60) of the augmented imaging information (401) .

6. Apparatus according to claim 5, wherein the display device (50) is at least one selected from: a three-dimensional display device; a holographic display device; an image projector; a head-worn display device.

7. Apparatus according to claim 6, wherein the display device comprises a projector (51a) configured to project an image into a physical working space, optionally a physical working space for the patient.

8. Apparatus according to any preceding claim, wherein the processing device (40) is configured to generate information relating to one or more of:
- a centreline within a body lumen;
- one or more walls of a body lumen;
- a junction in a body lumen;
- obstructions in a body lumen;
- calcification within a body lumen;
- a shape of a body lumen;
- contact force and/or pressure against a body lumen;
- contact force and/or pressure against an instrument;
- - deformation of the body in contact with an instrument;
- a position of an instrument within a body lumen;
- a native or artificial valve within a body lumen;
- navigation of an instrument within a body lumen;
- introduction of an instrument into a body lumen;
- incision by an instrument;
- an offset between an instrument and a centreline of a body lumen.

9. Apparatus according to any preceding claim, wherein the processing device (40) is configured to generate information relating to one or more of:
- one or more walls of a vascular lumen;
- a junction in a vascular lumen;
- a shape of a vascular lumen;
- one or more singular zones of a vascular lumen;
- one or more target areas for a medical instrument on a vascular wall and/or lumen.

10. Apparatus according to claim 9, wherein the processing device (40) is configured to generate information using pre-computed images of the patient and wherein the generated information is projected onto the visual display (60) and/or into the physical working space.

11. Apparatus according to any preceding claim, wherein the processing device is configured to generate information relating to at least one of:
- contact force and/or pressure against a body lumen; and
- contact force and/or pressure against an instrument.

12. Apparatus according to any preceding claim, wherein the processing device (40) is configured to generate warning information (51) about one or more hazard conditions along a lumen route.

13. Apparatus according to any preceding claim, wherein the processing device (40) is configured to generate navigation guidance (52) with respect to a lumen route to a target location.

14. Apparatus according to any preceding claim, wherein the medical imaging device (20) is an extracorporeal device.

15. Apparatus according to any preceding claim 1 to 12, wherein the medical imaging device (20) is at least one selected from: an intracorporeal device; an endoluminal device; an endovascular device.

16. Apparatus according to any preceding claim, wherein the second information comprise pre-procedural information obtained prior to a medical procedure.

17. Apparatus according to any preceding claim, wherein the second information comprises at least one selected from: x-ray image information; fluoroscopic image information; ct-scan image information; MRI image information; information from an instrument force sensor; information from an instrument pressure sensor; information from a magnetic resonance imaging device.

18. Apparatus according to any preceding claim, wherein the processing device (40) comprises an image processing engine (43) for correlating relative positioning between the first image information and the second information, to effect position registration (44) of the first and second image information.

19. Apparatus according to any preceding claim, further comprising at least one instrument (80) insertable at least partly into the body, and distinct from the medical imaging device (20).

20. Apparatus according to claim 18, further comprising robotic apparatus for manipulating the instrument (80).

21. A method of planning at least a portion of a medical procedure to be carried out by robot and/or remotely-operated apparatus, the planning method comprising:
operating a medical imaging device (20) comprising a non-ionizing imaging transducer, to generate first dynamic image information of a target area of a patient (70); and
electronically processing the first image information to generate augmented imaging (60) information by combining the first image information with second information from a source (30) different from the medical imaging device, the second information associated with at least one selected from: an instrument to be used during the procedure and/or the patient's anatomy.
